# EUROPEAN PATENT SPECIFICATION

(11) **EP 3 136 920 B1**
(45) Date of publication and mention of the grant of the patent: **14.02.2018**
(21) Application number: 15732938.4
(22) Date of filing: 28.04.2015
(51) Int. Cl.: A47K 1/00, A47K 5/12, A47K 10/48, A61L 2/00, A61L 2/22, A61L 9/00, B08B 3/10

(54) **A DEVICE FOR CLEANING HANDS**
EINE VORRICHTUNG ZUR REINIGUNG DER HÄNDE
UN DISPOSITIF DE LAVAGE DES MAINS

(30) Priority: 30.04.2014 IT MO20140117
(43) Date of publication of application: 08.03.2017
(73) Proprietor: Biomeco S.r.l., 42124 Reggio Emilia (IT)
(72) Inventor: STOPPONI, Marco, I-52021 Bucine (Arezzo) (IT)
(74) Representative: Gagliardelli, Fabrizio
(86) International application number: PCT/IB2015/053082
(87) International publication number: WO 2015/166406

(56) References cited:
- WO-A1-2011/092211
- US-A- 6 161 227
- US-A1- 2007 213 877
- US-A1- 2013 269 733
- US-A1- 2013 291 947
- US-B1- 6 431 189

## Description

The present invention has for an object a device for cleaning hands, as defined by claim 1.

A device for cleaning hands is known from document US6431189.

The device disclosed in this document comprises a closed case with a front opening, a plurality of nebuliser nozzles, a control unit, a sensor detecting the presence of hands insided the case, and a venturi valve for introducing a desinfectant.

In particular but not exclusively the device of the invention finds useful application in places where there is a high number of people who must clean or wash their hands such as for example in public premises, highway service stations and the like; the device herein disclosed, is also particularly useful in places where water resources are not abundant or where there is not provided any connection to a water distribution network.

On the base of statistical data, it was found that a normal cycle of hand cleaning or washing, usually comprising wetting, soaping and rinsing, requires about 2.5 liters of water. To reduce this consumption it has long been in use timed systems which allow the delivery of water only for a specified time (typically a few seconds). There are further used systems provided with photocells which allow the delivery of water only in the presence of the hands. Thanks to the systems like the ones above described, the water consumption is reduced to little more than half of the consumption indicated above (about 1.4 liters for every cleaning operation).

It is an aim of the present invention to provide a device that allow to obtain a thorough hands cleaning with an extremely limited use of water.

An advantage of the present invention is to provide a device enabling to obtain a thorough cleaning of the hands under ultra-clean conditions.

These aims and other advantages are all attained by the invention as defined in the appended claims.

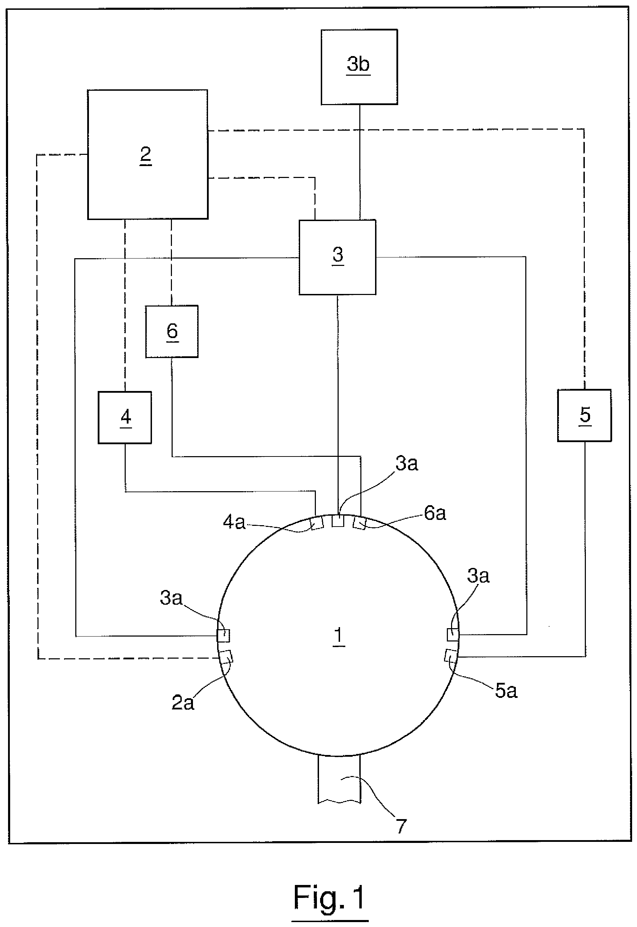

Further characteristics and advantages of the invention will become more apparent from the following description of the device, illustrated in the accompanying Figure 1 which shows a possible scheme of realization.

Likewise the methods of the known type, the not claimed method for cleaning hands herein disclosed, provides the use of water; however, unlike the methods of the prior art, the method of the present disclosure provides use of nebulised water for cleaning operations, which nebulised water is directed by command onto the hands to be cleaned.

The method to which the present disclosure relates, comprises a step of wetting hands with nebulised water; there is further provided a step of dispensing a detergent onto the hands to be cleaned and lastly a step of hand rinsing with nebulised water as well; in summary, the subject method involves the use of nebulised water in all steps which require water.

Conveniently there is provided a step of hand drying following the rinsing step; in this step there is afforded the use of drying air addressed onto the hands after rinsing thereof.

In order to make the action of nebulised water more effective, the not claimed method herein provides that the cleaning, particularly the supply of nebulised water, takes place within a constricted environment into which the hands to be cleaned are introduced. This is advantageous also due to the fact that it allows to use any drops of nebulised water which deposit on the walls of the constricted environment and fall onto the hands to be cleaned; a further advantage is that dispersion of detergent and drying air is prevented.

The not claimed method can also comprise a step of dispensing a disinfectant onto the hands which are placed within the constricted environment; this step, particularly useful in hospital environments, can be performed following cleaning operations or even as a separate step, upon request of the user.

In order to start the several steps afforded by the subject method, a step of detecting the presence of the hands within the constricted environment is provided; this step affords a command signal to activate hand cleaning operations and generally the various operations provided for by the method.

In order to implement the not claimed method herein disclosed, a device comprising a case 1 is used, which case 1 defines a constricted environment within which the hands to be cleaned are inserted. This case is, according to the invention as defined by claim 1, closed with a front opening so as to enable introduction of the hands; It can also be partially open on the top thereof, or supplied with an upper part at least partially transparent, so as to allow the user to see his hands during the several cleaning steps. The case 1 can also be realized in the form of a washbasin; with a case of this kind, the hands are introduced therein from above.

Typically in the lower part of the case there is provided a drain 7 which allows to drain the water as well as the other substances used during the cleaning operation.

The subject device comprises a plurality of nebuliser nozzles 3a which introduce into the case 1, by command, nebulised water sent to the nozzles by means of a pumping device 3; said nozzles are arranged in such a manner that the nebulised water is directed onto hands. The pumping device 3 sends water to the nozzles at a pressure between 10 and 15 bar. The water can be supplied to the pumping device from the water network or a reservoir 3b.

The device of the present invention comprises a first tap 4a which introduces into the case 1 by command, a detergent from a detergent tank 4; the device further comprising a second valve 6a which, by command, introduces into the case 1 a disinfectant coming from a disinfectant tank 6.

There is further provided at least one air diffuser 5a which introduces into the case 1, by command, drying air coming from a fan 5.

All the elements described hereinabove are widely known; positioning thereof relative to the case 1, depends on the case shape; such positioning will still be such as to allow to direct detergent, disinfectant and air onto the hands that are within the case.

In order for the various components of the device to be operated such as to perform the diverse steps of the not claimed method herein disclosed, there is afforded a sensor 2a that detects the presence of the hands within the case 1 and sends a command signal to a control unit 2, so as to activate cleaning operations. Normally the cleaning operations provide wetting the hands by activating the nozzles 3a a first time, dispensing detergent onto the hands by activating the first tap 4a, rinsing the hands by activating the nozzles 3a a second time, and finally drying the hands by activating the air diffuser 5a; the first three steps may be performed also by keeping the nozzles 3a continuously in operation. It takes about 20-25 seconds to perform above cleaning operations which allow a thorough cleaning of the hands with a consumption of water that is about 95% lower than that of the cleaning methods of the known type. Said cleaning operation start with detecting the presence of the hands within the case 1, and sequence and duration thereof are pre-set by the manufacturer of the device.

Furthermore, the subject device allows to clean the hands without that the user has the need to touch any taps, buttons, or any other control device; this aspect is particularly useful for ensuring hygiene during the whole cleaning operation.

The sequence of the operations described hereinabove was pre-set by the manufacturer and is therefore of the "standard" type; the device can be further supplied with a switch allowing the user to perform only some of the operations that the device is able to perform; by way of example, hands can be rinsed or disinfected by using one selector only; the latter operation is in fact often performed as a separate operation, for example in a hospital.

In figure 1 it is illustrated a diagram of the various components of the subject device. As stated above, said components may be arranged in any way; for example, they can all be contained within a single case, so as to provide a complete device, ready to be connected to the water network, the electrical distribution network, and the drain; it is instead possible to separate the various elements one from another, so as to facilitate the positioning thereof in places with limited space. It would also be possible to have a sort of dome, to which the various nozzles, taps and sensors are connected, to be placed over an existing washbasin.

Irrespective of the shape and size one wishes to give to the device in object, use of nozzles enabling to utilize nebulised water to perform cleaning operations shall not be disregarded.

## Claims

1. A device for cleaning hands, comprising: a closed case (1) that defines a constricted environment, having a front opening, into which environment the hands to be cleaned are inserted though the opening; a plurality of nebuliser nozzles (3a) that introduce into the case (1), by command, nebulised water, the water being sent to the nozzles by means of a pumping device (3);
a control unit;
a sensor (2a) that detects the presence of hands inside the case (1) and provides a command signal to said control unit (2) to activate the cleaning operations;
comprises a first tap (4a) that introduces into the case (1), by command, a detergent coming from a detergent tank (4);
a first air diffuser (5a) that introduces into the case (1), by command, drying air coming from a fan (5); and
a second tap (6a), being a valve, that introduces into the case (1), by command, a disinfectant coming from a disinfectant tank (6);
wherein said first tap (4a), said second tap (6a) and said air diffuser (5a) are such positioned so as to allow to direct detergent, disinfectant and air onto the hands that are within the case;
the control unit (2) being configured to activate the nozzles (3a) a first time, dispensing detergent onto the hands by activating the first tap (4a), rinsing the hands by activating the nozzles (3a) a second time, and drying the hands by activating the air diffuser (5a), said disinfectant being also dispensed onto the hands, activating said second tap (6a).

2. The device according to claim 1, **characterised in that** said pumping device (3) sends water to the nozzles at a pressure comprised between 10 and 15 bar.

## Patentansprüche

1. Vorrichtung zur Reinigung der Hände, umfassend: ein geschlossenes Gehäuse (1), das eine verengte Umgebung definiert, mit einer vorderen Öffnung, in die die zu reinigenden Hände durch die Öffnung eingeführt werden; eine Vielzahl von Verneblerdüsen (3a), die auf Befehl vernebeltes Wasser in das Gehäuse (1) einleiten, wobei das Wasser mittels einer Pumpvorrichtung (3) zu den Düsen geschickt wird;
eine Steuereinheit;
einen Sensor (2a), der das Vorhandensein von Händen innerhalb des Gehäuses (1) erfasst und ein Befehlssignal an die Steuereinheit (2) liefert, um die Reinigungsvorgänge zu aktivieren;
umfasst einen ersten Hahn (4a), der auf Befehl ein Reinigungsmittel, das von einem Reinigungsmitteltank (4) kommt, in das Gehäuse (1) einleitet; einen ersten Luftverteiler (5a), der auf Befehl Trocknungsluft, die von einem Gebläse (5) kommt, in das Gehäuse (1) einleitet; und
einen zweiten Hahn (6a), der ein Ventil ist, das auf Befehl ein Desinfektionsmittel, das aus einem Desinfektionstank (6) kommt, in das Gehäuse (1) einleitet;
wobei der erste Hahn (4a), der zweite Hahn (6a) und der Luftverteiler (5a) so positioniert sind, dass Reinigungsmittel, Desinfektionsmittel und Luft auf die Hände, die innerhalb des Gehäuses sind, gerichtet werden können; wobei die Steuereinheit (2) konfiguriert ist, um die Düsen (3a) ein erstes Mal zu aktivieren, wobei Reinigungsmittel durch Aktivieren des ersten Hahns (4a) auf die Hände abgegeben wird, wobei die Hände durch Aktivieren der Düsen (3a) ein zweites Mal abgespült werden und wobei die Hände durch Aktivieren des Luftverteilers (5a) getrocknet werden, wobei das Desinfektionsmittel ebenfalls auf die Hände durch Aktivieren des zweiten Hahns (6a) abgegeben wird,

2. Vorrichtung nach Anspruch 1, **dadurch gekennzeichnet, dass** die Pumpvorrichtung (3) Wasser mit einem Druck zwischen 10 und 15 bar zu den Düsen schickt.

## Revendications

1. Dispositif de lavage des mains, comprenant : une cavité fermée (1) définissant un environnement restreint, comportant une ouverture antérieure, environnement dans lequel les mains à laver sont insérées à travers l'ouverture ; une pluralité de buses de nébulisation (3a) introduisant dans la cavité (1), par commande, de l'eau nébulisée, l'eau étant envoyée aux buses au moyen d'un dispositif de pompage (3);
une unité de commande;
un capteur (2a) détectant la présence des mains à l'intérieur de la cavité (1) et fournissant un signal de commande à ladite unité de commande (2) pour activer les opérations de nettoyage;
comprenant un premier robinet (4a) introduisant dans la cavité (1), par commande, un détergent provenant d'un réservoir à détergent (4);
un premier diffuseur d'air (5a) introduisant dans la cavité (1), par commande, de l'air chaud provenant d'un ventilateur (5); et
un second robinet (6a), étant une vanne, introduisant dans la cavité (1), par commande, un désinfectant provenant d'un réservoir à désinfectant (6); dans lequel ledit premier robinet (4a), ledit second robinet (6a) et ledit diffuseur d'air (5a) sont positionnés de manière à permettre d'orienter le détergent, le désinfectant et l'air sur les mains étant à l'intérieur de la cavité;
l'unité de commande (2) étant configurée pour activer les buses (3a) une première fois, pour distribuer du détergent sur les mains en activant le premier robinet (4a), pour rincer les mains en activant les buses (3a) une seconde fois, et pour sécher les mains en activant le diffuseur d'air (5a), ledit désinfectant étant aussi distribué sur les mains en activant ledit second robinet (6a).

2. Dispositif selon la revendication 1, **caractérisé en ce que** ledit dispositif de pompage (3) envoie l'eau aux buses à une pression comprise entre 10 et 15 bars.
